# EUROPÄISCHE PATENTSCHRIFT

(11) **EP 1 978 873 B1**
(45) Veröffentlichungstag und Bekanntmachung des Hinweises auf die Patenterteilung: **01.10.2014**
(21) Anmeldenummer: 07703683.8
(22) Anmeldetag: 08.01.2007
(51) Int. Cl.: A61B 6/00

(54) **POSITIONIERVORRICHTUNG FÜR EIN MAMMOGRAFIEGERÄT**
POSITIONING DEVICE FOR A MAMMOGRAPHY UNIT
DISPOSITIF DE POSITIONNEMENT D'UN APPAREIL À MAMMOGRAPHIE

(30) Priorität: 03.02.2006 DE 102006005068
(43) Veröffentlichungstag der Anmeldung: 15.10.2008
(73) Patentinhaber: Siemens Aktiengesellschaft, 80333 München (DE)
(72) Erfinder: MEER, Oliver, 81371 München (DE); RAMSAUER, Martin, 90602 Pyrbaum (DE)
(86) Internationale Anmeldenummer: PCT/EP2007/050130
(87) Internationale Veröffentlichungsnummer: WO 2007/090693

(56) Entgegenhaltungen:
- WO-A-90/05485
- DE-A1- 10 353 611
- US-A- 5 018 176
- US-A1- 2005 100 129
- US-A1- 2005 113 681
- SIEMENS MEDICAL SOLUTIONS: "MAMMOMAT Novation Digital Mammography at its Very Best"[Online] 2004, XP002436875 Germany Gefunden im Internet: URL:http://www.medical.siemens.com/siemens /en_US/gg_sps_FBAs/files/brochures/MAMMOMA T_NovationDR/Mammomat_Novation_USA_Brochur e.pdf> [gefunden am 2007-06-08]

## Beschreibung

Die Erfindung betrifft eine Positioniervorrichtung für ein Mammografiegerät zur relativen Positionierung einer Strahlungsquelle und eines Objekthalters mit einem Geräteträgerabschnitt zur Aufnahme der Strahlungsquelle, mit einem Objektträgerabschnitt zur Aufnahme des Objekthalters, wobei der Objektträgerabschnitt über ein Verbindungselement mit dem Mammografiegerät verbindbar und/oder verbunden ist.

Mammografiegeräte führen Untersuchungen des Weichgewebes der menschlichen Brust mit Röntgenstrahlung durch und dienen insbesondere zur Früherkennung von Brustkrebs. Derartige Früherkennungsuntersuchungen gehören in einigen Ländern Europas bereits zu den Standardvorsorgeuntersuchungen, in Deutschland werden diese Untersuchungen zumindest vielfach durchgeführt. Aufgrund der Häufigkeit der Mammografie-Untersuchungen und dem gleichzeitigen Kostendruck im Gesundheitswesen ist es notwendig, die Handhabung der Mammografiegeräte zu vereinfachen und deren Anwendungsflexibilität zu erhöhen, um auf diese Weise Untersuchungszeit bei gleichbleibender oder verbesserter Untersuchungsqualität einzusparen.

Die Anmelderin vertreibt unter den Produktnamen "Mammomat 1000", "Mammomat 3000 Nova" und "Mammomat Novation" Mammografiegeräte, die in der konstruktiven Ausbildung ähnlich realisiert sind und stellvertretend am Beispiel des "Mammomat Novation" erläutert werden: Dieses Mammographiegerät weist einen Grundkörper und einen von dem Grundkörper auskragenden, abgewinkelten Gerätearm auf, an dessen freien Ende eine Strahlungsquelle angeordnet ist. Der Gerätearm ist als Blechkonstruktion realisiert und drehfest mit einer Drehachse des Mammographiegeräts verbunden, so dass die Strahlungsquelle um 360° um ein Isozentrum geschwenkt werden kann. Auf dem Gerätearm ist über eine Drehverbindung ein Objekttisch gelagert, der um 360° um das Isozentrum schwenkbar ist. Die Offenlegungsschrift DE 10353611 A1 offenbart ein weiteres Mammographiegerät.

Der Erfindung liegt die Aufgabe zugrunde, die Robustheit und damit die Messgenauigkeit eines Mammographiegeräts zu verbessern.

Diese Aufgabe wird erfindungsgemäß durch eine Positioniervorrichtung mit den Merkmalen des Anspruchs 1 gelöst. Vorteilhafte und/oder bevorzugte Ausführungsformen sind in den Unteransprüchen beansprucht.

Die erfindungsgemäße Positioniervorrichtung ist geeignet und/oder ausgebildet für ein Mammografiegerät und dient zur relativen Positionierung einer Strahlungsquelle und eines Objekthalters. Die Strahlungsquelle ist bevorzugt zur Emission von weicher Röntgenstrahlung im Bereich von unter 50 kV (Kilovolt), insbesondere unter 30 kV ausgebildet. Der Objekthalter ist vorzugsweise zur Aufnahme einer weiblichen Brust - nachfolgend auch als Untersuchungsobjekt bezeichnet - ausgebildet, wobei der Objekthalter insbesondere eine Einrichtung zur Kompression der Brust aufweist. Der Objekthalter umfasst üblicherweise einen Objekttisch und eine hiergegen verschiebliche Kompressionsplatte. Zudem sind ein Geräteträgerabschnitt und ein Objektträgerabschnitt vorgesehen, die zur Aufnahme der Strahlungsquelle bzw. des Objekthalters ausgebildet sind. Der Objektträgerabschnitt ist über ein Verbindungselement mit dem Mammografiegerät verbindbar und/oder verbunden.

Erfindungsgemäß ist der Geräteträgerabschnitt relativ zu dem Verbindungselement drehbar, insbesondere relativ zu dem Objektträgerabschnitt und/oder dem Mammografiegerät gelagert. Insbesondere ist der Geräteträgerabschnitt unabhängig und/oder entkoppelt von dem Objektträgerabschnitt drehbar gelagert. Vorzugsweise ist der Geräteträgerabschnitt auf dem Verbindungselement drehbar gelagert.

Die Erfindung geht dabei von der Überlegung aus, dass es bei den aus dem Stand der Technik bekannten Positioniervorrichtungen nachteilig ist, den Objektträgerabschnitt auf einer mit dem Geräteträgerabschnitt fest verbundenen Drehachse zu lagern, da bei Untersuchungen, bei denen verschiedene Einstrahlungswinkel der Röntgenstrahlung erforderlich sind, eine Änderung der Position des Geräteträgerabschnitts zu einer Dejustage des Objektträgerabschnitts führen kann.

Es ist daher vorgesehen, die bekannte offene kinematische Kette, bei der der Objektträgerabschnitt auf dem Geräteträgerabschnitt gelagert ist, abschnittsweise umzubilden und den Geräteträgerabschnitt relativ drehbar zu dem Verbindungselement zwischen Mammografiegerät und Objektträgerabschnitt zu lagern. Auf diese Weise ist es möglich, das Untersuchungsobjekt von verschiedenen Einstrahlrichtungen zu bestrahlen, ohne bei der Lageänderung des Geräteträgerabschnitts die Lage des Objektträgerabschnitts und somit des Untersuchungsobjekts zu verändern.

Bei einer bevorzugten Weiterbildung ist der Geräteträgerabschnitt als Gerätearm ausgebildet und weist ein freies sowie ein an dem Verbindungselement angelenktes Ende auf. Das freie Ende ist zur Aufnahme der Strahlungsquelle sowie gegebenenfalls weiterer Komponenten, wie z.B. Blende, Elektronik etc., ausgebildet. Das angelenkte Ende umschließt das Verbindungselement zumindest abschnittsweise und/oder weist eine Durchgangs- oder Aufnahmeöffnung auf, durch die das Verbindungselement zumindest abschnittsweise ragt. Durch diese konstruktive Ausführung wird der Hebelarm von Mammografiegerät zur Strahlungsquelle in zwei Abschnitte geteilt, wobei der erste Abschnitt durch das Verbindungselement und der zweite Abschnitt durch den Geräteabschnitt gebildet wird. Bevorzugt weist das Verbindungselement in einem Bereich, auf dem der Geräteträgerabschnitt gelagert und/oder abgestützt ist, einen Durchmesser von mindestens 10 cm, bevorzugte mindestens 15 cm, insbesondere mindestens 20 cm auf. Diese Ausbildungen unterstützen eine sehr kompakte Realisierung des Geräteträgerabschnitts, so dass die Positioniervorrichtung in sich stabil und insbesondere wenig anfällig hinsichtlich Schwingungen der Strahlungsquelle ist.

Bevorzugt ist an dem angelenkten Ende ein Gegengewicht zu dem Gerätearm angeordnet, insbesondere derart, dass ein Gegenmoment zu dem Gerätearm gebildet wird. Gerätearm und Gegengewicht sind hinsichtlich der Durchgangs- oder Aufnahmeöffnung vorzugsweise diametral zueinander angeordnet.

Erfindungsgemäß ist das angelenkte Ende als ein ring- oder torusartiger Körper ausgebildet, der die Durchgangs- oder Aufnahmeöffnung bildet.

Bei einer vorteilhaften Weiterbildung der Positioniervorrichtung ist das Verbindungselement als Dreh- und/oder Schwenkachse für den Objektträgerabschnitt ausgebildet und erlaubt somit eine Rotation des Objektträgerabschnitts. Hierzu ist der Objektträgerabschnitt vorzugsweise mit der Dreh- und/oder Schwenkachse drehfest verbunden und rotiert gemeinsam mit dieser oder ist drehbar auf der insbesondere feststehenden Dreh- und/oder SchwenkAchse gelagert.

Der Objektträgerabschnitt umfasst einen zweiten Körper, der als Deckelement ausgebildet ist und auf dem das Verbindungselement, insbesondere auf der Dreh- und/oder Schwenkachse, sitzt. Alternativ ist der zweite Körper ring- oder torusartig ausgebildet. Bevorzugt ist der erste Körper zwischen dem Mammografiegerät und dem zweiten Körper angeordnet und/oder wird beim Betrieb der Positioniervorrichtung entsprechend angeordnet. Diese Ausbildung birgt den Vorteil, dass bei einer Rotation der Strahlungsquelle während einer Untersuchung diese von der Patientin aus gesehen hinter dem zweiten Körper dreht und durch den ersten Körper.verdeckt ist. Eine Quetschgefahr für Bediener und Patientin wird auf diese Weise minimiert.

Insbesondere zur weiteren Minimierung der Quetschgefahr ist vorgesehen, dass der erste und der zweite Körper in Richtung der Längserstreckung des Verbindungselements zumindest abschnittsweise deckungsgleich und/oder senkrecht zur Längserstreckung bündig miteinander abschließend ausgebildet sind. Bevorzugt bilden erster und zweiter Körper gemeinsam eine geschlossene, insbesondere kreisförmige Außenkontur.

Erfindungsgemäß bleibt die deckungsgleiche.und/oder bündig miteinander abschließende Anordnung der beiden Körper auch bei einer Rotation von Geräteträgerabschnitt und/oder Objektträgerabschnitt erhalten.

Bei einer besonders vorteilhaften Ausführungsform sind Geräteträgerabschnitt und Objektträgerabschnitt drehbar um eine gemeinsame Drehachse angeordnet, wobei die gemeinsame Drehachse insbesondere parallel zu der Längserstreckung des Verbindungselements angeordnet ist.

Die gemeinsame Drehachse führt bevorzugt durch ein gemeinsames Isozentrum, welches im Zentrum des späteren, zu Untersuchungszwecken positionierten Untersuchungsobjektes angeordnet ist. Strahlungsquelle sowie Objekthalter sind somit derart drehbar gelagert, dass auch bei Änderung des Einstrahlungswinkels der Strahlungsquelle und/oder des Anstellwinkels des Objekthalters die Position des Untersuchungsobjekts nicht verändert werden muss. Diese isozentrische Anordnung von Geräteträgerabschnitt und Objektträgerabschnitt bietet den Vorteil, dass das Mammografiegerät nur einmalig für den Patienten eingestellt werden muss.

Bei einer bevorzugten Weiterbildung der Positioniervorrichtung ist der Geräteträgerabschnitt relativ zu dem Objektträgerabschnitt um bis zu +/- 25° verschwenkbar. Dieser Schwenkwinkel erlaubt die Änderung des Einstrahlungswinkels für Stereotaxie-Untersuchungen, bei denen meist Schwenkwinkel von +/- 10° bzw. +/- 15°zweckmäßig sind. Die Positioniervorrichtung erlaubt aber auch Tomosynthese-Untersuchungen, bei denen ein kontinuierliches Verschwenken der Strahlungsquelle von bis zu +/-25° zur Erzeugung einer tomographie-ähnlichen Aufnahme des Untersuchungsobjekts notwendig sein können. Insbesondere bei der Stereotaxie als auch bei der Tomosynthese ist es vorteilhaft, wenn der Geräteträgerabschnitt unabhängig oder entkoppelt von dem Objektträgerabschnitt drehbar oder schwenkbar gelagert ist.

Bevorzugt erlaubt das als Dreh- oder Schwenkachse ausgebildete Verbindungselement eine Drehung bzw. Schwenkung des Objektträgerabschnitts von bis zu +/-180°. Diese Ausbildung ermöglicht die Aufnahme des Untersuchungsobjekts in Standarddarstellungen wie z.B. einer cranio-caudalen (CC) oder mediolateralen-obliquen (MLO) Darstellung.

Zur individuellen Anpassung des Mammografiegeräts an die jeweilige Patientin ist vorgesehen, dass das Verbindungselement und somit der Geräteträgerabschnitt und der Objektträgerabschnitt relativ zu dem Mammografiegerät in der Höhe und/oder in der seitlichen Lage verstellbar ausgebildet ist. Insbesondere die Höhenverstellung erlaubt es, das Mammografiegerät komfortabel an verschiedene Körpergrößen von Patientinnen anzupassen.

Bei einer bevorzugten Ausführungsform der Positioniervorrichtung sind auf dem Objektträgerabschnitt und/oder dem Geräteträgerabschnitt, insbesondere auf den ersten und/oder zweiten Körper, Displays, Bedienelemente, Hebel etc. angeordnet. Aufgrund einer optionalen konzentrischen Anordnung der Bedienelemente auf dem ersten bzw. dem zweiten Körper kann aus al-, len üblichen Positionen oder Einstellungen (ML, MLO, etc.) der Positioniervorrichtung komfortabel auf die Bedienelemente etc. zugegriffen werden.

Die Einstellung der Positioniervorrichtung, insbesondere die Rotation oder Schwenkung des Geräteträgerabschnitts und/oder des Objektträgerabschnitts, erfolgt entweder manuell oder motorisch. Die beschriebene Positioniervorrichtung ist besonders geeignet für ein Diagnostizierverfahren zur Röntgenuntersuchung eines Untersuchungsobjekts, insbesondere zur Mammografie.

Nachfolgend wird ein Ausführungsbeispiel der Erfindung anhand von Zeichnungen näher erläutert. Dabei zeigen:
- FIG 1: ein Ausführungsbeispiel einer Positioniervorrichtung als Teil eines Mammografiegeräts in einer modellhaften 3D-Darstellung;
- FIG 2: das Ausführungsbeispiel in FIG 1 reduziert auf eine schematische Darstellung eines Aktorikabschnitts zur Illustration der möglichen Freiheitsgrade der Aktorik;
- FIG 3a,b,c: das Mammografiegerät in FIG 1 in drei Momentaufnahmen in verschiedenen Positionen oder Einstellungen der Positioniervorrichtung;
- FIG 4a,b: Ausschnittsvergrößerungen der Positioniervorrichtung zur besseren Darstellung von integrierten Bedienelementem auf einem ringartigen "Doughnutabschnitt" der Positioniervorrichtung.

Einander entsprechende Teile sind in allen Figuren stets mit denselben Bezugszeichen versehen.

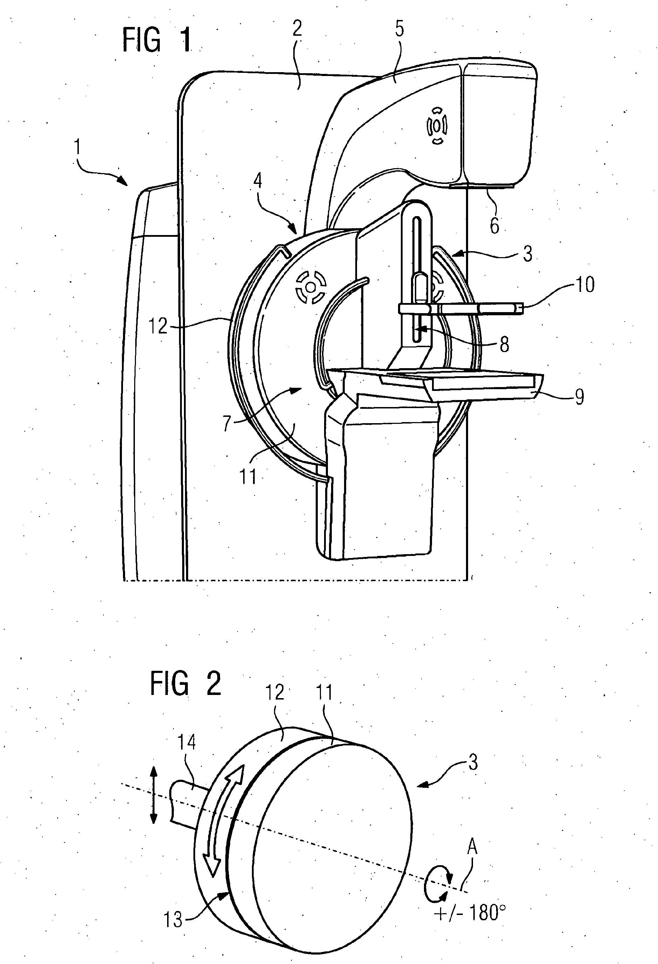

FIG 1 zeigt eine schematische dreidimensionale Ansicht eines Mammografiegerätes 1 schräg von der Seite. Das Mammografiegerät 1 umfasst einen Grundkörper 2 sowie eine Positioniervorrichtung 3, die von dem Grundkörper 2 getragen wird.

Die Positioniervorrichtung 3 weist einen Geräteträgerabschnitt 4 auf, an dem über einen abgewinkelten Gerätearm 5 eine Strahlungsquelle 6 sowie weitere, nicht gezeigte KomponenTen, wie z. B. Elektronik, Blende etc., fest angebracht sind.

Ferner umfasst die Positioniervorrichtung 3 einen Objektträgerabschnitt 7, der mit einem Objekthalter 8 fest verbunden ist. Der Objekthalter 8 weist eine Auflageplatte 9 zur Auflage des Untersuchungsobjekts, insbesondere einer weiblichen Brust, sowie eine Andruckplatte 10 auf, die parallel zur Auflageplatte 9 angeordnet ist. Die Andruckplatte 10 ist relativ zu der Auflageplatte 9 verschiebbar, so dass das Untersuchungsobjekt komprimiert werden kann. Optional wird auf dem Objektträgerabschnitt 7 eine Biopsievorrichtung (nicht gezeigt) zur Feinnadel- oder Stanzbiopsie angeordnet, welche z.B. mit der Auflageplatte 9 fest verbunden ist, und insbesondere eine Positioniervorrichtung für die Feinnadel bzw. das Stanzteil aufweist. Der Objektträgerabschnitt 7 umfasst weiterhin als zweiten Körper einen tellerförmigen Deckelabschnitt 11, der parallel zur Vorderseite des Grundkörpers 2 angeordnet ist, und in der Projektion auf den Grundkörper 2 eine kreisrunde Außenkontur aufweist. Der Deckelabschnitt 11 ist über eine Drehachse 14 (FIG 2) mit dem Grundkörper 2 verbunden. Je nach Ausführungsform der Positioniervorrichtung 3 ist der Deckelabschnitt 11 drehfest mit der Drehachse 14 gekoppelt oder ist auf dieser drehbar gelagert. Die Drehbewegung des Deckelabschnitts 11 erfolgt um eine Achse A (FIG 2), die parallel zu der Drehachse 14 (FIG 2) ausgerichtet ist und durch ein Isozentrum des Mammografiegeräts 1 führt. Das Isozentrum liegt an der Position des späteren Mittelpunkts des Untersuchungsobjekts, beispielsweise ca. 3 cm oberhalb der Auflageplatte 9. Objekthalter 8 und Deckelabschnitt 11 sind drehfest miteinander verbunden, so dass eine Drehung des Deckelabschnitts 11 zu einer Drehung des Objekthalters 8 führt.

Der Geräteträgerabschnitt 4 umfasst als ersten Körper einen hinteren Ringkörper 12, der fest mit dem Gerätearm 5 verbunden ist und drehbar auf der Drehachse 14 (FIG 2) gelagert ist. Deckelabschnitt 11 und hinterer Ringkörper 12 sind deckungsgleich zueinander angeordnet und schließen seitlich bündig ab, so dass die Gefahr von Quetschungen bei Drehung des Geräteträgerabschnitts 4 oder des Objektträgerabschnitts 7 vermindert wird. Der Außendurchmesser von Deckelabschnitt 11 und/oder hinterem Ringkörper 12 entspricht in etwa dem radialen Abstand zwischen Isozentrum und Oberkante des Objekthalters 8. Der Abstand zwischen Isozentrum und Strahlungsquelle 6 wird somit ungefähr zur Hälfte durch den hinteren Ringkörper 12 und zur anderen Hälfte durch den Gerätearm 5 überbrückt, so dass der Gerätearm 5 vergleichsweise kurz und kompakt und somit schwingungssicher ausgeführt ist. Ausgehend von der nicht gezeigten Achse A können Deckelabschnitt 11 und/oder hinterer Ringkörper 12 in azimutaler Richtung eine gleichmäßige Gewichtsverteilung aufweisen. Alternativ kann insbesondere der hintere Ringköper 12 diametral zu dem Gerätearm 5 ein Gegengewicht aufweisen, so dass ein Gegenmoment zu dem Gerätearm 5 gebildet ist.

Die FIG 2 zeigt die Positioniervorrichtung 3 in FIG 1 in einer stark abstrahierten 3D-Darstellung schräg von der Seite. In dieser Darstellung sind nur der Deckelabschnitt 11 sowie der hintere Ringkörper 12 gezeigt, wobei zwischen diesen eine Trennebene 13 ausgebildet ist. Der Deckelabschnitt 11 und der hintere Ringkörper 12 können mittels der Drehachse 14 um die Achse A um +/- 180° gedreht werden, die in der Mittelachse der Drehachse 14 liegt. Der hintere Ringkörper 12 ist in einem Bereich von +/- 25° relativ und entkoppelt zu dem vorderen Ringkörper 11 ebenfalls um die Achse A drehbar gelagert. Die gesamte Positioniervorrichtung 3 ist höhenverstellbar in dem Grundkörper 2 aufgenommen, wobei die Achse A bzw. die Drehachse 14 in Richtung des gezeigten Doppelpfeils, also in der Höhe, verstellbar ist. An dem Deckelabschnitt 11 sind - wie bereits in Zusammenhang mit FIG 1 erläutert - der Objekthalter 8 sowie Griffe, Displays angeordnet und evtl. eine Biopsieeinheit und/oder Vergrößerungstische befestigbar. An dem hinteren Ringkörper 12 ist der Gerätearm 5 und somit insbesondere die Strahlungsquelle 6 befestigbar. Deckelabschnitt 11 und hinterer Ringkörper 12 bilden zusammen einen stufenlosen Zylinder- oder Kegelmantel, so dass insbesondere im Bereich der Trennebene 13 keine Quetschbereiche vorhanden sind.

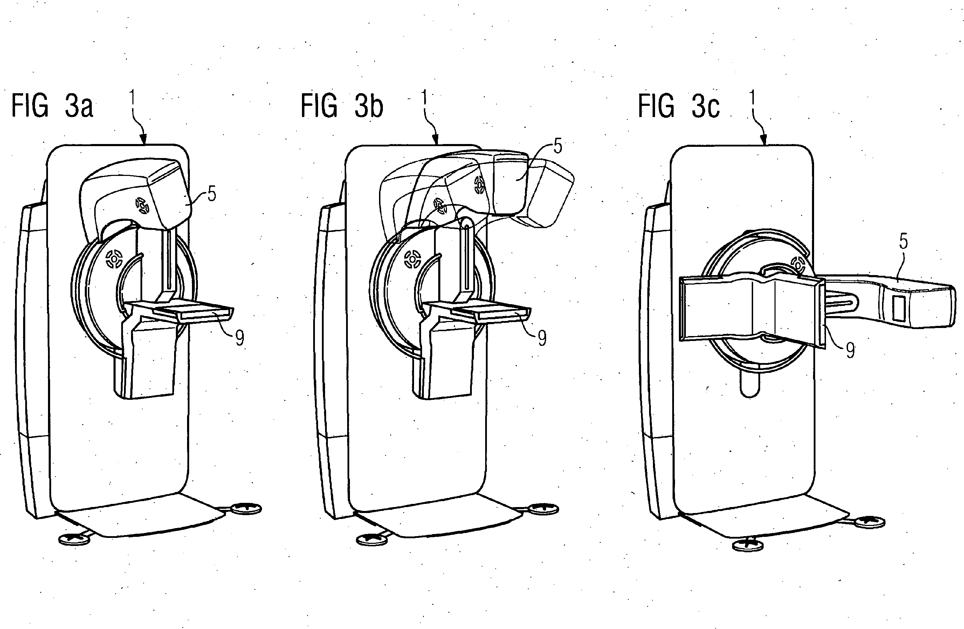

Die FIG 3 a,b und c zeigen das Mammografiegerät 1 in FIG 1 jeweils in einer Momentaufnahme während verschiedener Untersuchungsmethoden. Die FIG 3a zeigt das Mammografiegerät 1 mit einer wagerecht ausgerichteten Auflageplatte 9 und einen um ca. -15 ° gegenüber der Senkrechten verschwenkten Gerätearm 5 während einer Stereotaxie-Untersuchung. Die Verschwenkung des Gerätearms 5 erfolgt um das Isozentrum.

Die FIG 3b illustriert eine Tomosynthese-Untersuchung mit dem Mammografiegerät 1 in FIG.1. Bei dieser Untersuchungsmethode wird der Geratearm 5 mit der Strahlungsquelle 6 kontinuierlich in einem Winkelbereich von -25° bis +25°, insbesondere automatisch verschwenkt, während gleichzeitig der Objekthalter 8 und insbesondere die Auflageplatte 9 stationär verbleibt. Durch diese Aufnahmetechnik ist es möglich eine tomographieähnliche Aufnahme des Untersuchungsobjekts zu erhalten.

Die FIG 3c zeigt das Mammografiegerät 1 in FIG 1 schließlich in einer Einstellung, bei der die Auflageplatte 9 senkrecht ausgerichtet ist und der Gerätearm 5 sowie die Strahlungsquelle.6 ebenfalls um +90° gegenüber der Senkrechten verschwenkt sind. Auch ausgehend von dieser Einstellung ist es möglich, den Gerätearm 5 relativ zum Objekthalter 8 und Auflageplatte 9 um bis zu +/- 25° zu verschwenken.

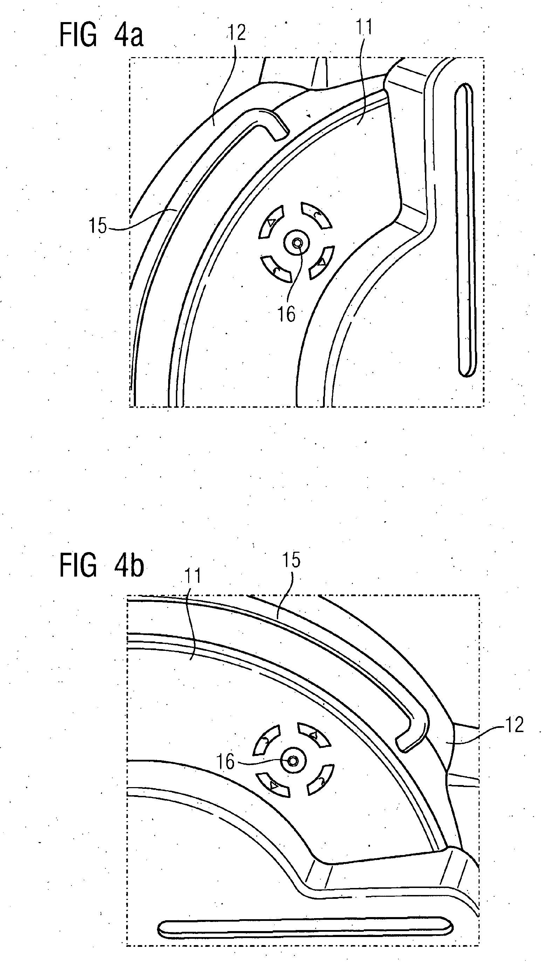

Die FIG 4a, b zeigen jeweils einen Detailausschnitt des Mammografiegeräts 1 in FIG 1. Diese Detailausschnitte zeigen jeweils Griffe 15 sowie Bedienknöpfe 16, die an dem vorderen Ringkörper 11 angebracht sind und beispielsweise die Funktionen "Gerät auf/ab", "Gerätedrehung links/rechts", "Licht ein/aus" umfassen. Die Bedienelemente 16 befinden sich auf der Stirnseite des vorderen Ringkörpers 12, wohingegen die Griffe 15 z.B. an dessen radialer Außenseite angeordnet sind. Die Griffe 15 können auch anderweitig, beispielsweise radial bzw. konzentrisch angeordnet sein. Von allen üblichen Positionen (ML, MLO, etc.) der Positioniervorrichtung 3 kann aufgrund der konzentrischen Anordnung der Bedienelemente 16 einfach auf diese zugegriffen werden.

## Patentansprüche

1. Positioniervorrichtung (3) für ein Mammografiegerät (1) zur relativen Positionierung einer Strahlungsquelle (6) und eines Objekthalters (8)
mit einem Geräteträgerabschnitt (4) zur Aufnahme der Strahlungsquelle (6),
mit einem Objektträgerabschnitt (7) zur Aufnahme des Objekthalters (8),
wobei der Objektträgerabschnitt (7) über ein Verbindungselement (14) mit dem Mammografiegerät (1) verbindbar und/oder verbunden ist,
wobei der Geräteträgerabschnitt (4) relativ zu dem Verbindungselement (14) und gegenüber dem Objektträgerabschnitt (4) drehbar gelagert ist,
wobei der Geräteträgerabschnitt (7) einen Gerätearm (5) umfasst, wobei dessen freies Ende zur Aufnahme der Strahlungsquelle (6) ausgebildet ist und dessen angelenktes Ende eine Durchgangsöffnung aufweist, durch die das Verbindungselement (14) zumindest abschnittsweise ragt, und/oder dessen angelenktes Ende das Verbindungselement (14) umschließt, **dadurch gekennzeichnet daß** das angelenkte Ende als ein erster ringartiger Körper (12) ausgebildet ist,
der Objektträgerabschnitt (7) einen zweiten ringförmigen Körper (11) umfasst, der einen Deckelabschnitt zur Abdeckung des ersten Körpers (12) bildet, und
der erste Körper (12) und der zweite Körper (11) deckungsgleich sind, so dass die Umfangsflächen des ersten und zweiten Körpers (12, 11) miteinander unabhängig von der Rotationsstellung des Geräteträgerabschnitts (4) relativ zu dem Objektträgerabschnitt (7) fluchten.

2. Positioniereinrichtung (3) nach Anspruch 1,
wobei das Verbindungselement als Drehachse (14) ausgebildet ist.

3. Positioniervorrichtung (3) nach Anspruch 1 oder 2,
wobei der erste Körper (12) zwischen dem Mammografiegerät (1) und dem zweiten Körper (11) angeordnet ist.

4. Positioniervorrichtung (3) nach einem der vorhergehenden Ansprüche,
wobei Geräteträgerabschnitt (4) und Objektträgerabschnitt (7) um ein gemeinsames Zentrum drehbar angeordnet sind.

5. Positioniereinrichtung (3) nach einem der vorhergehenden Ansprüche,
wobei der Geräteträgerabschnitt (4) relativ zu dem Objektträgerabschnitt (7) um bis zu +/- 25° verschwenkbar ist.

6. Positioniereinrichtung (3) nach einem der vorhergehenden Ansprüche,
wobei die Drehachse oder Schwenkachse (14) eine Drehung des Objektträgerabschnitts (4) von bis zu +/- 180° ermöglicht.

7. Positioniereinrichtung (3) nach einem der vorhergehenden Ansprüche,
wobei das Verbindungselement (14) relativ zu dem Mammografiegerät (1) in der Höhe und/oder in der seitlichen Lage verstellbar ausgebildet ist.

8. Positioniereinrichtung (3) nach einem der vorhergehenden Ansprüche,
wobei auf dem Objektträgerabschnitt (7) und/oder Geräteträgerabschnitt (4) Displays, Griffe (15), Bedienknöpfe (16) etc. angeordnet sind.

## Claims

1. Positioning device (3) for a mammography device (1) for the relative positioning of a radiation source (6) and an object holder (8)
with a device support section (4) for accommodating the radiation source (6),
with an object support section (7) for accommodating the object holder (8),
with the object support section (7) being connectable and/or connected to the mammography device (1) by means of a connecting element (14),
with the device support section (4) being mounted such as to be able to rotate relative to the connecting element (14) and with respect to the object support section (4),
with the device support section (7) including a device arm (5), with the free end of which being embodied to accommodate the radiation source (6) and the hinged end of which having a through opening, through which the connecting element (14) protrudes at least in sections and/or the hinged end of which surrounds the connecting element (14), **characterised in that** the hinged end is embodied as a first annular body (12),the object support section (7) includes a second ring-shaped body (11), which forms a cover section for covering the first body (12), and
the first body (12) and the second body (11) are congruent, so that the peripheral surfaces of the first and second bodies (12, 11) align with one another independently of the rotation position of the device support section (4) relative to the object support section (7).

2. Positioning device (3) according to claim 1, wherein the connecting element is embodied as an axis of rotation (14).

3. Positioning device (3) according to claim 1 or 2, wherein the first body (12) is arranged between the mammography device (1) and the second body (11).

4. Positioning device (3) according to one of the preceding claims, wherein the device support section (4) and object support section (7) are rotatably arranged about a common centre.

5. Positioning device (3) according to one of the preceding claims, wherein the device support section (4) can be pivoted about up to +/- 25° relative to the object support section (7).

6. Positioning device (3) according to one of the preceding claims, wherein the axis of rotation or swivel axis (14) enables a rotation of the object support section (4) of up to +/- 180°.

7. Positioning device (3) according to one of the preceding claims, wherein the connecting element (14) is embodied to be adjustable relative to the mammography unit (1) in terms of its height and/or lateral position.

8. Positioning device (3) according to one of the preceding claims, wherein displays, handles (15), control buttons (16) etc. are arranged on the object support section (7) and/or device support section (4).

## Revendications

1. Dispositif de positionnement (3) d'un appareil de mammographie (1) pour le positionnement relatif d'une source de rayonnement (6) et d'un support d'objet (8),
avec une partie porte-appareil (4) destinée à recevoir la source de rayonnement (6),
avec une partie porte-objet (7) destinée à recevoir le support d'objet (8),
la partie porte-objet (7) étant reliée ou pouvant être reliée à l'appareil de mammographie (1) au moyen d'un élément de liaison (14),
la partie porte-appareil (4) étant montée en rotation par rapport à l'élément de liaison (14) et par rapport à la partie porte-appareil (4),
la partie porte-appareil (7) comprenant un bras (5), dont l'extrémité libre est conçue pour recevoir la source de rayonnement (6) et dont l'extrémité articulée présente une ouverture de passage que l'élément de liaison (14) traverse au moins en partie, et/ou dont l'extrémité articulée entoure l'élément de liaison (14), **caractérisé en ce que**
l'extrémité articulée est conçue comme un premier corps annulaire (12),
la partie porte-objet (7) comprend un second corps annulaire (11) qui forme une partie couvercle destinée à recouvrir le premier corps (12), et
le premier corps (12) et le second corps (11) sont identiques de sorte que les surfaces périphériques du premier et second corps (12, 11) sont alignées entre elles quelle que soit la position de rotation de la partie porte-appareil (4) par rapport à la partie porte-objet (7).

2. Dispositif de positionnement (3) selon la revendication 1, l'élément de liaison étant conçu comme axe de rotation (14).

3. Dispositif de positionnement (3) selon la revendication 1 ou 2,
le premier corps (12) étant disposé entre l'appareil de mammographie (1) et le second corps (11).

4. Dispositif de positionnement (3) selon l'une des revendications précédentes,
les parties porte-appareil (4) et porte-objet (7) étant montées en rotation autour d'un centre commun.

5. Dispositif de positionnement (3) selon l'une des revendications précédentes,
la partie porte-appareil (4) pouvant pivoter par rapport à la partie porte-objet (7) jusqu'à +/- 25°.

6. Dispositif de positionnement (3) selon l'une des revendications précédentes,
l'axe de rotation ou axe de pivotement (14) permettant une rotation de la partie porte-objet (4) jusqu'à +/- 180°.

7. Dispositif de positionnement (3) selon l'une des revendications précédentes,
l'élément de liaison (14) étant conçu de manière à pouvoir être réglé en hauteur et/ou en position latérale par rapport à l'appareil de mammographie (1).

8. Dispositif de positionnement (3) selon l'une des revendications précédentes,
sur la partie porte-objet (7) et/ou la partie porte-appareil (4) étant disposés des écrans, poignées (15), boutons de commande (16), etc.
